# EUROPEAN PATENT SPECIFICATION

(11) **EP 0 998 304 B1**
(45) Date of publication and mention of the grant of the patent: **20.08.2003**
(21) Application number: 98930755.8
(22) Date of filing: 27.05.1998
(51) Int. Cl.: A61K 47/48, A61K 9/14, A61K 9/48

(54) **PHARMACEUTICAL COMPOSITIONS COMPRISING A BASIC DRUG, A CYCLODEXTRIN, A POLYMER AND AN ACID**
ARZNEIMITTEL ENTHALTEND EINEN BASISCHEN WIRKSTOFF, EIN CYCLODEXTRIN, EIN POLYMER UND EINE SAURE
COMPOSITIONS PHARMACEUTIQUES COMPRENANT UN MEDICAMENT BASIQUE, UNE CYCLODEXTRINE, UN POLYMERE ET UN ACIDE

(30) Priority: 05.06.1997 GB 9711643
(43) Date of publication of application: 10.05.2000
(73) Proprietor: JANSSEN PHARMACEUTICA N.V., 2340 Beerse (BE)
(72) Inventor: VANDECRUYS, Roger, Petrus, Gerebern, 2260 Wesreloo (BE)
(74) Representative: Quaghebeur, Luc
(86) International application number: EP9803189
(87) International publication number: WO98055148

(56) References cited:
- EP-A- 0 300 526
- EP-A- 0 472 327
- EP-A- 0 614 666
- EP-A- 0 689 844
- WO-A-90/14082
- WO-A-94/12217
- WO-A-95/00144
- WO-A-97/18245
- US-A- 4 351 846
- US-A- 4 956 351
- US-A- 5 206 025
- US-A- 5 472 954

## Description

This invention relates to pharmaceutical compositions and dosage forms providing improved drug release and uptake on administration into externally voiding body cavities (e.g. the gi tract) or on topical administration, for acid solubilized basic drug compounds.

Many drug compounds, while possessing desired therapeutic properties, are used inefficiently due to their poor water solubilities. Thus for example where such compounds are administered orally, only a small fraction of the drug is taken up into the blood during transit of the gi tract. As a result, to achieve adequate drug uptake it may be necessary to administer high doses of the drug compound, to prolong the period of drug administration or to make frequent administrations of the drug compound. Indeed, the poor solubility and hence poor bioavailability of a drug may cause an alternative drug, perhaps one with undesired side effects or one which requires invasive administration (e.g. by injection or infusion), to be used in place of the poorly soluble drug.

One approach to poor solubility is to derivatise the drug molecule to introduce water solubilizing groups, e.g. ionic groups such as carboxyl groups or non-ionic groups such as polyhydroxyalkyl groups, so as to produce a more soluble derivative. This approach however is not always successful as it may not be possible to maintain adequately high therapeutic efficacy and adequately low toxicity or other side effects. Thus one example of a poorly water soluble drug which has not been superseded by a solubilized derivative is the antifungal agent itraconazole.
Attempts have therefore been made to enhance the uptake of drugs such as itraconazole by increasing the surface area of the drug compound exposed to saliva or gastric fluid, and hence promote dissolution of the drug compound, by thinly coating the drug compound onto essentially inert carrier particles, e.g. sugar beads. This however has the drawback that the volume of solid composition required to administer a given quantity of the drug compound is quite high since the carrier contributes significantly to the overall administration volume. Since administration of large volume capsules or tablets, or of large quantities of smaller volume capsules or tablets, provides difficulties for the patient, the drawbacks of this approach are obvious.

Yet another approach has been to administer the drug compound in the form of a solution of the drug compound and a drug complexing agent such as a cyclodextrin. This approach has limitations also in that the dosage volume is constrained by the solubilizing power of the complexing agent, readily unitized solid dosage forms can not be used, and there is no gradual release of the drug compound for biological uptake.

EP-A-0,689,844 describes complexes of vinpocetine formed with cyclodextrin and pharmaceutical compositions containing them.

WO 94/12217 relates to pharmaceutical compositions comprising a therapeutic agent, a carboxy-containing polymer and cyclodextrin in an aqueous medium.

However, we have now found that by combining such drug compounds with a cyclodextrin, a water-soluble acid from 35 to 95 % by weight and a water-soluble organic polymer, an administration form may be produced which surprisingly improves the biological uptake of the drug compound, in particular a form which can surprisingly improve the time profile for the drug content of the plasma of the patient (*i.e*. the pharmacokinetic profile defined by such parameters as AUC, tₘₐₓ, Cₘₐₓ, etc.).

Thus viewed from one aspect the invention provides a pharmaceutical composition comprising a no more than sparingly water-soluble basic drug compound, a cyclodextrin, a physiologically tolerable water-soluble acid, and a physiologically tolerable water-soluble organic polymer characterized in that it comprises from 35 to 95 % by weight of acid relative to the total weight of cyclodextrin, drug compound, organic polymer and acid.
Viewed from a further aspect the invention provides the use of a no more than sparingly water-soluble basic drug compound, a cyclodextrin, a physiologically tolerable water-soluble acid from 35 to 95 % by weight (relative to the total weight of cyclodextrin, drug compound, organic polymer and acid), and a physiologically tolerable water-soluble organic polymer for the manufacture of a pharmaceutical composition according to the invention for use in a method of therapy or diagnosis of the human or non-human animal (e.g. mammalian, reptilian or avian) body.

The compositions of the invention may if desired be aqueous, but in general will preferably be substantially water-free, e.g. containing up to 3% by weight, preferably less than 1% by weight water, and most preferably less than 0.5% water, but may be mixed with water immediately before administration or may be coated and dispersed in an aqueous medium whereby the coating is only broken down after administration. Such aqueous compositions are deemed to fall within the scope of the invention.

Depending on the selection of components, the compositions of the invention may be liquid, solid or semi-solid - e.g. gel-like. Preferably the compositions are non-freeflowing at ambient temperature (e.g. 21°C), other than as free flowing particulates. Thus the compositions at ambient temperature are preferably solids or semi-solids or, less preferably, highly viscous fluids.

In the compositions of the invention the drug compound, acid, cyclodextrin and organic polymer are intimately admixed.

Thus where the composition is particulate, the acid, drug compound, cyclodextrin and organic polymer are mixed together within the particles (e.g. at the molecular level following solvent removal from a solution of these components). Granulate mixtures where individual particles do not contain all four components, or have cores of one or more components coated with other components are not preferred. This intimate admixture is important since the effects of the components are complimentary at the microscopic level during dissolution of the compositions of the invention.

Preferably, all components are dispersed so as to form a system that is chemically and physically uniform or homogenous throughout, or consists of one phase as defined in thermodynamics ; such a dispersion will be called a glass thermoplastic phase or system hereinafter. The components of the glass thermoplastic system are readily bioavalaible to the organisms to which they are administered. This advantage can probably be explained by the ease with which said glass thermoplastic system can form liquid solutions when contacted with a body liquid such as gastric juice. The case of dissolution may be attributed at least in part to the fact that the energy required for dissolution of the components from a glass thermoplastic system is less than that required for the dissolution of components from a crystalline or microcrystalline solid phase.

As the cyclodextrin in the compositions of the invention, there may be used any of the physiologically tolerable water-soluble substituted or unsubstituted cyclodextrins or physiologically tolerable derivatives thereof, e.g. α-, β- or γ-cyclodextrins or derivatives thereof, in particular derivatives wherein one or more of the hydroxy groups are substituted, e.g. by alkyl, hydroxyalkyl, carboxyalkyl, alkylcarbonyl, carboxyalkoxyalkyl, alkylcarbonyloxyalkyl, alkoxycarbonylalkyl or hydroxy-(mono or polyalkoxy)alkyl groups, wherein each alkyl or alkylene moiety preferably contains up to six carbons.

Substituted cyclodextrins which can be used in the invention include polyethers, e.g. as described in US Patent 3,459,731. In general, to produce these, unstibstituted cyclodextrins are reacted with an alkylene oxide, preferably under superatmospheric pressure and at an elevated temperature, in the presence of an alkaline catalyst. Since a hydroxy moiety of the cyclodextrin can be substituted by an alkylene oxide which itself can react with yet another molecule of alkylene oxide, the average molar substitution (MS) is used as a measure of the average number of moles of the substituting agent per glucose unit. The MS can be greater than 3 and theoretically has no limit. In the cyclodextrin derivatives for use in the compositions according to the present invention the M.S. is conveniently in the range of 0.125 to 10, in particular of 0.3 to 3, or from 0.3 to 1.5. Preferably the M.S. ranges from about 0.3 to about 0.8, in particular from about 0.35 to about 0.5 and most particularly it is about 0.4. M.S. values determined by NMR or IR preferably range from 0.3 to 1, in particular from 0.55 to 0.75.

Further examples of substituted cyclodextrins include ethers wherein the hydrogen of one or more cyclodextrin hydroxy groups is replaced by C₁₋₆alkyl, hydroxyC₁₋₆-alkyl, carboxy-C₁₋₆alkyl or C₁₋₆alkyloxycarbonyl-C₁₋₆alkyl groups or mixed ethers thereof. In particular such substituted cyclodextrins are ethers wherein the hydrogen of one or more cyclodextrin hydroxy groups is replaced by C₁₋₃alkyl, hydroxy-C₂₋₄alkyl or carboxy-C₁₋₂alkyl or more particularly by methyl, ethyl, hydroxyethyl, hydroxypropyl, hydroxybutyl, carboxymethyl or carboxyethyl.

In the foregoing definitions, the term "C₁₋₆alkyl" is meant to include straight and branched saturated hydrocarbon radicals, having from 1 to 6 carbon atoms, such as methyl, ethyl 1-methylethyl, 1,1-dimethylethyl, propyl, 2-methylpropyl, butyl, pentyl, hexyl and the like.

Such ethers can be prepared by reacting a cyclodextrin with an appropriate O-alkylating agent or a mixture of such agents in a concentration selected such that the desired cyclodextrin ether is obtained. The reaction is preferably conducted in a solvent in the presence of a base. With such ethers, the degree on substitution (DS) is the average number of substituted hydroxy functions per glucose unit, the DS being thus 3 or less.

In the cyclodextrin derivatives for use in the compositions according to the present invention, the DS preferably is in the range of 0.125 to 3, in particular 0.3 to 2, more particularly 0.3 to 1, and the MS is in the range of 0.125 to 10, in particular 0.3 to 3 and more particularly 0.3 to 1.5.

Of particular utility in the present invention are the β-cyclodextrin ethers, e.g. dimethyl-β-cyclodextrin as described in Drugs of the Future, Vol. 9, No. 8, p. 577-578 by M. Nogradi (1984) and polyethers, e.g. hydroxypropyl-β-cyclodextrin and hydroxyethyl-β-cyclodextrin. Such alkyl ethers may for example be methyl ethers with a degree of substitution of about 0.125 to 3, e.g. about 0.3 to 2. Such a hydroxypropyl cyclodextrin may for example he formed from the reaction between β-cyclodextrin and propylene oxide and may have a MS value of about 0.125 to 10, e.g. about 0.3 to 3.

Especially suitable cyclodextrins are β-CD, 2,6-dimethyl-β-CD, 2-hydroxyethyl-β-CD. 2-hydroxyethyl-γ-CD, 2-hydroxypropyl-γ-CD and (2-carboxymethoxy)propyl-β-CD, and in particular 2-hydroxypropyl-β-CD.

Besides simple cyclodextrins, branched cyclodextrins and cyclodextrin polymers may also be used.

Other cyclodextrins are described for example in Chemical and Pharmaceutical Bulletin 28: 1552-1558 (1980), Yakugyo Jiho No. 6452 (28 March 1983), Angew. Chem. Int. Ed. Engl. 19: 344-362 (1980), US-3,459,731, EP-A-0,149,197. EP-A-0,197,571, US-4,535,152, WO-90/12035 and GB-2,189,245. Other references describing cyclodextrins for use in the compositions according to the present invention, and which provide a guide for the preparation, purification and analysis of cyclodextrins include the following: "Cyclodextrin Technology" by József Szejtli, Kluwer Academic Publishers (1988) in the chapter Cyclodextrins in Pharmaceuticals; "Cyclodextrin Chemistry" by M.L. Bender et al., Springer-Verlag, Berlin (1978); "Advances in Carbohydrate Chemistry", Vol. 12, Ed. by M.L. Wolfrom. Academic Press, New York in the chapter The Schardinger Dextrins by Dexter French at p. 189-260: "Cyclodextrins and their Inclusion Complexes" by J. Szejtli, Akademiai Kiado, Budapest, Hungary (1982): I. Tabushi in Acc. Chem. Research, 1982, 15, p. 66-72; W. Sanger, Angewandte Chemie, 92, p. 343-361 (1981): A.P. Croft and R.A. Bartsch in Tetrahedron, 39. p. 1417-1474 (1983); Irie et al. Pharmaceutical Research, 5, p. 713-716, (1988): Pitha et al. Int. J. Pharm. 29, 73, (1986); DE 3,118.218; DE-3,317,064: EP-A-94,157; US-4,659,696; and US-4,383,992.

More recent examples of substituted cyclodextrins include sulfobutylcyclodextrins (US-5,134,127-A). Their use is also envisaged in the present invention.

The cyclodextrin used is preferably a β-cyclodextrin, in particular hydroxypropyl-β-cyclodextrin. The most preferred cyclodextrin derivative for use in the compositions of the present invention is hydroxypropyl-β-cyclodextrin having a M.S. in the range of from 0.35 to 0.50 and containing less than 1.5% unsubstituted β-cyclodextrin. M.S. values determined by NMR or IR preferably range from 0.55 to 0.75.

Nevertheless, the choice of cyclodextrin may be directed by the ability of the selected drug compound to be complexed by a particular cyclodextrin - thus the cyclodextrins with greater affinity for the particular drug compound may be preferred.

In the compositions of the invention, the cyclodextrin is preferably present at 5 to 70% by weight, more preferably 8 to 55%, most preferably 10 to 45% by weight (relative to the total weight of cyclodextrin, acid, organic polymer and drug). The quantity of cyclodextrin used however will generally be dependent on the quantity of drug and the molar ratio of cyclodextrin to drug will preferably lie in the range 100:1 to 1:5, especially 50:1 to 1:2, more especially 10:1 to 1:1.

The acid used in the compositions of the invention may be any of the water-soluble physiologically tolerable acids, in particular any of the inorganic or, more preferably, organic acids conventionally used in the preparation of acid salts of drug compounds, e.g. citric, fumaric, tartaric, maleic, malic, succinic, oxalic, malonic, benzoic, mandelic and ascorbic acids.

Tartaric acid and more especially citric acid are preferred since the salts they form with drug compounds usually have a reduced tendency to precipitate from aqueous solutions. In general however, any acid which is not so strong as to cause degradation of the cyclodextrin and yet which is capable, on the addition of water, of generating a low pH environment, preferably lower than pH 4 and ideally about pH 2, may be used. The acid may be in liquid (e.g. solution) or solid form; however acids which are solid at ambient conditions in their anhydrous or hydrate forms will generally be preferred.

In the compositions of the invention, the acid is present from 35 to 95% by weight, preferably 35 to 60% by weight (relative to the total weight of cyclodextrin, drug compound, organic polymer and acid). The amount of acid used will be dependent upon the selected acid and drug compound, but in general an increase in the relative proportion of acid will result in an acceleration of drug dissolution on contact with water. The amount of acid used will normally be at least the amount necessary to form a 1:1 salt with the drug compound.

In general, the acid will form a significant proportion of dosage forms that dissolve rapidly in body fluids. Typically, they will comprise from 50 to 95% by weight of acid, preferably 50 to 90% by weight, more preferably 55 to 60% by weight. Thus viewed from a further aspect the invention provides a pharmaceutical composition comprising an organic drug compound, a water-soluble physiologically tolerable acid, a water-soluble physiologically tolerable cyclodextrin and a water-soluble physiologically tolerable organic polymer, characterised in that the weight ratios of drug compound to acid and of drug compound to cyclodextrin are no more than 2:1, preferably no more than 1.5:1, especially preferably no more than 1:1, and particularly preferably no more than 0.9:1, especially no more than 0.5:1.

The organic polymer used in the compositions of the invention may be any of the physiologically tolerable water soluble synthetic, semi-synthetic or non-synthetic organic polymers.

Thus for example the polymer may be a natural polymer such as a polysaccharide or polypeptide or a derivative thereof, or a synthetic polymer such as a polyalkylene oxide (e.g. PEG), polyacrylate, polyvinylpyrrolidone, etc. Mixed polymers, e.g. block copolymers and glycopeptides may of course be used.

It is believed that the effect of the organic polymer arises from an enhancement in viscosity which serves to stabilize supersaturated solutions of the drug compound on dissolution of the composition of the invention. Thus the polymer conveniently has a molecular weight in the range 500D to 2 MD, and conveniently has an apparent viscosity of 1 to 100 mPa.s when in a 2% aqueous solution at 20°C. For example, the water-soluble polymer can be selected from the group comprising
- alkylcelluloses such as methylcellulose,
- hydroxyakylcelluloses such as hydroxymethylcellulose, hydroxyethylcellulose, hydroxypropylcellulose and hydroxybutylcellulose,
- hydroxyalkyl alkylcelluloses such as hydroxyethyl methylcellulose and hydroxypropyl methylcellulose,
- carboxyalkylcelluloses such as carboxymethylcellulose,
- alkali metal salts of carboxyalkylcelluloses such as sodium carboxymethylcellulose,
- carboxyalkylalkylcelluloses such as carboxymethylethylcellulose,
- carboxyalkylcellulose esters,
- starches,
- pectins such as sodium carboxymethylamylopectin,
- chitin derivates such as chitosan,
- heparin and heparinoids,
- polysaccharides such as alginic acid, alkali metal and ammonium salts thereof, carrageenans, galactomannans, tragacanth, agar-agar, gum arabic, guargum and xanthan gum,
- polyacrylic acids and the salts thereof,
- polymethacrylic acids and the salts thereof, methacrylate copolymers,
- polyvinylalcohol,
- polyvinylpyrrolidone, copolymers of polyvinylpyrrolidone with vinyl acetate,
- polyalkylene oxides such as polyethylene oxide and polypropylene oxide and copolymers of ethylene oxide and propylene oxide, e.g. poloxamers and poloxamines.

Non-enumerated polymers which are pharmaceutically acceptable and have appropriate physico-chemical properties as defined hereinbefore are equally suited for preparing compositions according to the present invention.

Particularly preferably the organic polymer is a cellulose ether, e.g. methyl cellulose, hydroxyethylmethylcellulose, or hydroxypropylmethylcellulose (HPMC), for example a Methocel® (available from Colorcon, England) such as Methocel A, Methocel E, Methocel F, Methocel K, Methocel J or Methocel HB or a Metolose® such as Metolose SM, Metolose SH or Metolose SE. Especially preferably the organic polymer is a hydroxypropylmethylcellulose, e.g. from 5 cps Methocel E to 15000 cps Methocel K15M.

Even very small quantities of the organic polymer serve to achieve a beneficial effect in the compositions of the invention. Thus in the compositions of the invention the organic polymer is conveniently present at 0.05 to 35% by weight, preferably 0.1 to 20%, more preferably 0.5 to 15%, and most preferably 2 to 11% by weight (relative to the total weight of drug compound, acid, cyclodextrin and organic polymer). The content and viscosity grade of the organic polymer both affect the dissolution profile for the drug compound in the compositions of the invention, with increased organic polymer content and/or increased viscosity grade (e.g. 15000 mPa.s in place of 5 mPa.s (mPa.s values being at 2% aqueous solution at 20°)) both tending to decelerate drug compound dissolution). Accordingly the selection of the identity and quantity of the organic polymer will generally depend upon the dissolution profile that is desired. For example, a composition that provides sustained release of the drug, will comprise a water soluble polymer having an apparent viscosity of more than 1,000 mPa.s when dissolved in a 2% aqueous solution at 20°C.

The drug compound used in the compositions of the invention may be any organic or inorganic basic material which is no more than sparingly soluble, *i.e*. which is sparingly soluble, slightly soluble, very slightly soluble, or practically insoluble in pure water at 21°C (ie. requiring from 30, from 100, from 1000 or from 10000 parts water to put 1 part by weight drug compound into solution).

Examples of such poorly water-soluble compounds that may be used in the compositions of the invention include nifedipine, itraconazole (described in EP-A-6711), saperconazole (see US-A-4916134), (-)-[2S-[2α,4α(S*)]]-4-[4-[4-[4-[[2-(4-chlorophenyl]-2-[[(4-methyl-4*H*-1,2,4-triazol-3-yl)thio]methyl]-1,3-dioxolan-4-yl]methoxy]phenyl]-1-piperazinyl]phenyl]-2,4-dihydro-2-(1-methylpropyl)-3*H*-1,2,4-triazol-3-one (Compound 40 in WO96/13499), cisapride (described in EP-A-76530), (B)-N-[4-[2-ethyl-1-(1H-1,2,4-triazol-1-yl)butyl]phenyl]-2-benzothiazolamine (described in WO-97/49704); methyl 6,11-dihydro-11-[1-[2-[4-(2-quinolinylmethoxy)phenyl]ethyl]-4-piperidinylidene]-5*H*-imidazo[2,1-b][3]benzazepine-3-carboxylate (described in WO-97/34897);
4-[[4-amino-6-[(2,6-dichlorophenyl)methyl]-1,3,5-triazin-2-yl]amino]benzonitrile (described in EP-0,834,507);
(B-cis)-1-[4-[4-[4-[[4-(2,4-difluorophenyl)-4-(1*H*-1,2,4-triazol-1-ylmethyl)-1,3-dioxolan-2-yl]methoxy]phenyl]-1-piperazinyl]phenyl]-3-(1-methylethyl)-2-imidazolidinone;
(2S-cis)-1-[4-[4-[4-[[4-(2,4-difluorophenyl)-4-(1*H*-1,2,4-triazol-1-ylmethyl)-1,3-dioxolan-2-yl]methoxy]phenyl]-1-piperazinyl]phenyl]-3-(1-methylethyl)-2-imidazolidinone;
3-[2-[3,4-dihydrobenzofuro[3,2-c]pyridin-2(1*H*)-yl]ethyl]-2-methyl-4*H*-pyrido-[1,2-a]pyrimidin-4-one;
*N*-[2-[4-(4-chlorophenyl)-1-piperazinyl]ethyl]-2-benzothiazolamine;
(B1)-*N*-[4-[2-(dimethylamino)-1-(1*H*-imidazol-1-yl)propyl]phenyl]-2-benzo-thiazolamine (described in WO-97/49704)
(B)-6-[amino(4-chlorophenyl)(1-methyl-1*H*-imidazol-5-yl)methyl]-4-(3-chlorophenyl)-1-methyl-2(1*H*)-quinolinone;
(B)-*N*-[4-[2-ethyl-1-(1*H*-1,2,4-triazol-1-yl)butyl]phenyl]-2-benzothiazolamine; 3-[6-(dimethylamino)-4-methyl-3-pyridinyl]-2,5-dimethyl-*N,N*-dipropylpyrazolo-[2,3-a]pyrimidin-7-amine monohydrochloride;
(S)-[1-[2-[3-[(2,3-dihydro-1*H*-inden-2-yl)oxy]-4-methoxyphenyl]propyl]-1*H*-imidazol-2-yl]cyanamide; and
(+)-(B-trans)-4-[1-[3,5-bis(trifluoromethyl)benzoyl]-2-(phenylmethyl)-4-piperidinyl]-*N*-(2,6-dimethylphenyl)-1-piperazineacetamide (S)-hydroxybutanedioate (1:1).

Further suitable basic active ingredients are those which exert a local physiological effect, as well as those which exert a systemic effect, either after penetrating the mucosa or - in the case of oral administration - alter transport to the gastro-intestinal tract with saliva. The dosage forms prepared from the compositions according to the present invention are particularly suitable for active ingredients which exert their activity during an extended period of time, i.e. drugs having a half-life of at least several hours. Examples thereof include those of the following active agents that have basic character:
analgesic and anti-inflammatory drugs (celecoxib, MK966, L-745,337, NSAIDs, fentanyl, indomethacin, ketoprofen, nabumetone, oxyphenbutazone, paracetamol. phenylbutazone, piroxicam, tramadol) ; anti-arrhythmic drugs (gallopamil, procainamide, quinidine, verapamil) ; antibacterial and antiprotozoal agents (amoxicillin, ampicillin, benzathine penicillin, benzylpenicillin, cefaclor, cefadroxil, cefprozil, cefuroxime axetil, cephalexin, chloramphenicol, chloroquine, ciprofloxacin, clarithromycin, clavulanic acid, clindamycin, doxyxycline, erythromycin, flucloxacillin, halofantrine, isoniazid, kanamycin, lincomycin, mefloquine, minocycline, nafcillin, neomycin, norfloxacin, ofloxacin, oxacillin, phenoxymethyl-penicillin, pyrimethamine-sulfadoxime, quinine, streptomycin); anti-coagulants (warfarin) ; antidepressants (amitriptyline, amoxapine, butriptyline, clomipramine, desipramine, dothiepin, doxepin, fluoxetine, fluvoxamine, gepirone, imipramine, lithium carbonate, mianserin, milnacipran, nortriptyline, paroxetine, sertraline ; 3-[2-[3,4-dihydrobenzofuro[3,2-c]pyridin-2(1*H*)-yl]ethyl]-2-methyl-4*H*-pyrido[1,2-a]pyrimidin-4-one) ; anti-diabetic drugs (glibenclamide, metformin) : antiepileptic drugs (carbamazepine, clonazepam, ethosuximide, phenobarbitone, phenytoin, primidone, topiramate, valpromide) ; antifungal agents (amphotericin, clotrimazole, econazole, fluconazole, flucytosine, griseofulvin, itraconazole, ketoconazole, miconazole nitrate, nystatin, terbinafine, voriconazole) ; antigout (benzbromarone, probenecid) ; antihistamines (astemizole, cinnarizine, cyproheptadine, decarboethoxyloratadine, fexofenadine, flunarizine, levocabastine, loratadine, norastemizole, oxatomide, promethazine, terfenadine) : anti-hypertensive drugs (captopril, clonidine, cyclizine, diazoxide, dihydralazine, enalapril, fosinopril, guanethidine, ketanserin, lisinopril, minoxidil, prazosin, ramipril, rescinnamine, reserpine, terazosin) ; anti-muscarinic agents (atropine sulphate, hyoscine) ; antivirals (acyclovir, AZT, ddC, ddI, ganciclovir, loviride, tivirapine, 3TC, delavirdine, indinavir, nelfinavir, ritonavir, saquinavir) ; antineoplastic agents and antimetabolites (adriamycine, cladrihine, dactinomycin, daunorubicin, doxorubicin, etoposide, mitomycin, mitoxantrone, paclitaxel, taxol, taxotere, trimetrexate, vincristine, vinblasline) ; anti-migraine drugs (alniditan, naratriptan, sumatriptan) ; anti-Parkinsonian drugs (bromocryptine mesylate, carbidopa, levodopa, selegiline) : antipsychotic, hypnotic, anxiolylic and sedating agents (alprazolam, buspirone, chlordiazepoxide, chlorpromazine, chlorprothixene, clozapine, diazepam, flupenthixol, fluphenazine, flurazepam, haloperidol, 9-hydroxyrisperidonc, lorazepam, mazapertine, melperone, methaqualone, olanzapine, oxazepam, pimozide, pipamperone, piracetam, promazine, risperidone, selfotel, seroquel, sertindole, sulphide, temazepam, thioridazine, thiothixene, triazolam, trifluoperazine, trifluperidol, triflupromazine, ziprasidone, zolpidem) ; anti-stroke agents (lubeluzole, lubeluzole oxide, riluzole, aptiganel, eliprodil, remacemide) ; antitussive (dextromethorphan, laevodropropizine, noscapine) ; beta-adrenoceptor blocking agents (atenolol, bupranolol, carvedilol, labetalol, metipranolol, metoprolol, nebivolol, oxprenolol, propanolol) ; cardiac inotropic agents (amrinone, digitoxin, digoxin, milrinone) ; corticosteroids (beclomethasone dipropionate, betamethasone, budesonide, cortisone, dexamethasone, fludrocortisone, hydrocortisone, methylprednisolone, paramethasone, prednisolone, prednisone, triamcinolone) ; disinfectants (chlorhexidine) ; diuretics (acetazolamide, amiloride, benzthiazide, chlorothiazide, chlorthalidone, dichlorphenamide, ethacrynic acid, ethoxzolamide, frusemide, hydrochlorothiazide, hydroflumethiazide, isosorbide, polythiazide, spironolactone, triamterene, trichloromethiazide) ; enzymes ; ergot alkaloids (codergocrine, ergotamine, nicergolin) ; essential oils (anethole, anise oil, caraway, cardamom, cassia oil, cineole, cinnamon oil. clove oil, coriander oil, dementholised mint oil, dill oil, eucalyptus oil, eugenol, ginger, lemon oil, mustard oil, neroli oil, nutmeg oil, orange oil, peppermint, sage, spearmint, terpineol, thyme) ; gastro-intestinal agents (bromopride, cimetidine, cisapride, elebopride, diphenoxylate, domperidone, famotidine, lansoprazole, loperamide, loperamide oxide, mesalazine, metoclopramide, mosapride, nizatidine, norcisapride, olsalazine, omeprazole, pantoprazole, perprazole, pirenzepine, prucalopride, ranitidine, rabeprazole, ridogrel, sulphasalazine) ; haemostatics (aminocaproic acid) ; immunosuppressants (cyclosporin A, tacrolimus) ; lipid regulating agents (atorvastatin, lovastatin, pravastatin, probucol, simvastalin) ; local anaesthetics (benzocaine, lignocaine) ; opioid analgesics (buprenorphine, codeine, dextromoramide, dextropropoxyphene, dihydrocodeine, hydrocodone, oxycodone, morphine, papaverine, pentazoeine, pethidine) ; parasympathomimetics (eptastigmine, galanthamine, metrifonate, neostigmine, physostigmine, tacrine, donepezil, rivastigmine, milameline, sabcomeline, talsaclidine, xanomcline, memantine, lazabemide) ; sex hormones (androgens : methyltestosterone, oxymetholone, stanozolol ; oestrogens : conjugated oestrogens, ethinyloestradiol, mestranol, oestradiol, oestriol, oestrone ; progestogens ; chlormadinone acetate, cyproterone acetate, 17-deacetyl norgestimate, desogestrel, dienogest, dydrogesterone, ethynodiol diacetate, gestodene, 3-keto desogestrel, levonorgestrel, lynestrenol, medroxy-progesterone acetate, megestrol, norethindrone, norethindrone acetate, norethisterone, norethisterone acetate, norethynodrel, norgestimale, norgestrel, norgestrienone, progesterone, quingestanol acetate) ; stimulating agents (sildenafil) ; sympathomimetics (ephedrine, clenbuterol, fenoterol, norfenefrine, pseudoephedrine) ; vasodilators (amlodipine, amyl nitrite, buflomedil, buphenine, carbocromen, diltiazem, dipyridamole, glyceryl trinitrate, isosorbide dinitrate, lidoflazine, molsidomine, nicardipine, nifedipine, nimodipine, oxpentifylline, pentaerythritol tetranitrate).

Other examples include those of the following active agents that have basic character:

| | | |
|---|---|---|
| alpha-Lipoic acid | lactose | methylxanthine |
| 8-Methoxypsoralen | lithium salts | phytomenadione |
| Allopurinol | magnesium salts | propylthiouracil |
| alpha.-Tocopherol | menadione | |
| iron salts | methylthiouracil | |

Basic drug compounds suitable for use in the compositions of the invention include drugs of all types conventionally administered topically (e.g. in a gel patch) or into an externally voiding body duct, e.g. orally, nasally, aurally, rectally or vaginally. Such drugs include in particular antifungals, calcium channel blockers, antibacterials, antihypertensives, antivirals, analgesics, apolipoprotein B synthesis inhibitors, and drugs which modify transit of gi tract contents (e.g. antidiarrhoea agents or motility promoters). Indeed, the invention is particularly applicable to poorly water-soluble imidazole, triazole, imidazo-benzazepines, nitrophenyl-pyridine, *N,N*'-bisphenyl-piperazine, and *N*-phenoxyalkyl-piperidine derivatives, e.g. the compounds mentioned above and compounds as described in EP-A-6711, WO96/13499 and EP-A-76530.

The compositions of the invention may conveniently contain the drug compound at 0.001 to 50% by weight, preferably 0.1 to 35%, more preferably 0.5 to 30%, especially 8 to 25% and most especially 10 to 15% by weight (relative to the total weight of acid, cyclodextrin, organic polymer and drug compound). The quantity of drug will of course depend upon the desired dissolution profile, the intrinsic solubility of the drug compound and the drug dosage required where the drug is to be delivered in dosage units (e.g. capsules, coated tablets, etc).

Thus the present invention also provides pharmaceutical dosage forms comprising a therapeutically effective amount of a composition as described hereinbefore.

For example if the drug is to be delivered in a standard capsule (e.g. with a 900 mg capacity for a glass thermoplastic system as described in the Examples hereto, and the desired drug dose is 100 mg/capsule) then the quantities and natures of the other composition components may be selected to give the desired drug dissolution profile - in general only a small quantity of organic polymer, e.g. 20 to 50 mg, may be necessary, and the balance may be made up from acid and cyclodextrin with the ratio of acid to cyclodextrin being set according to the required dissolution profile, e.g. with 200 to 400 mg cyclodextrin and 450 to 650 mg acid.

Besides the drug compound, the organic polymer, the acid and the cyclodextrin, the compositions of the invention may contain other conventional pharmaceutical excipients, e.g. flavours, colouring agents, antioxidants, bulking agents, fats, waxes, coating agents, dispersants, suspension fluids (e.g. where the composition coated with a gastric juice resistant coating and dispersed as particles in a suspension fluid such as water or a syrup), etc. Preferably such components when in intimate admixture with the drug compound will make up only a minor proportion of the composition, e.g. 0.01 to 10% by weight (relative to the total weight of acid, organic polymer, cyclodextrin and drug compound). However where the composition of the invention is encapsulated or disposed in a carrier (e.g. a fluid or a solid or semi-solid matrix), the further components not in intimate admixture with the drug compound (e.g. coating or encapsulating materials, dispersion media, etc.) may of course make up a minor or major proportion, e.g. 5 to 95% by weight, of the overall composition.

The compositions of the invention may be prepared by making an intimate admixture of the drug compound, cyclodextrin, acid and organic polymer. This may be effected most straightforwardly by dissolving these components in a liquid solvent therefor and subsequently removing the solvent. Thus viewed from a further aspect the invention provides a process for the preparation of a pharmaceutical composition, said process comprising: dissolving a drug compound, a water-soluble cyclodextrin, a physiologically tolerable water-soluble acid and a physiologically tolerable water-soluble organic polymer in a solvent; removing solvent from the resultant solution: optionally forming the resultant product into desired shapes; and optionally coating the resulting product with a physiologically tolerable coating material.

The solvent used in the process of the invention is preferably a physiologically tolerable material. suitably an organic solvent such as a C₁₋₆ alkanol (e.g. ethanol), acetone, DMF, a linear or cyclic ether (e.g. diethyl ether, dimethyl ether, or THF), cyclohexane, DMSO, etc. or a solvent mixture that also may comprise water. For an acid with a high melting point, solvents or solvent mixtures which have high boiling points may conveniently be used; generally however the boiling point of the solvent or solvent system will be no more than about 100°C. Such solvents may be used efficiently in the production of the compositions of the invention and the level of residual solvent will be minimal. The solvent may conveniently be removed by evaporation, e.g. under reduced pressure, and as this may leave some solvent residue (e.g. up to 3% by weight) it is particularly desirable to use a solvent such as ethanol (or an ethanol-water mixture) which is a permitted pharmaceutical excipient.

If the drug compound is insoluble or poorly soluble in the solvent of choice, the process of the invention may involve dispersion of microparticles (e.g. nanoparticles having a particle size of 1 to 100 nm) of the drug compound in the solvent rather than full dissolution of the drug compound. If this is done, it is desirable that the drug compound particles be as small as possible. Nanoparticles of insoluble compounds may be prepared for example by various precipitation techniques or by milling with physiologically tolerable inorganic beads, e.g. of zirconia (EP-0,499.299).

The solvent removal may be essentially complete or it may he incomplete, in the former case to produce a solid or a gel-like solid or semi-solid, and in the latter case to produce a viscous fluid which can for example be filled into capsules.

In general, essentially complete solvent removal will he preferred as the resultant product can then readily be shaped. Shaping may be effected by spray-drying the solution (to provide the product in particulate form), by evaporation of Solvent from solution disposed in molds, by molding (e.g. injection molding), by extrusion and the like. In general the product can be formed when hot and allowed to solidify on cooling. The shaped product may likewise be produced in film or sheet form by evaporation or by pouring a heated mass onto a plate and evaporating off the solvent.

In one preferred embodiment the product is shaped by filling into (e.g. by pouring or by extrusion) capsule shells, e.g. of gelatin.

The product may be hygroscopic, and thus may be "tacky" if touched by hand due to its absorption of moisture from the skin. Accordingly it is particularly preferred for the product to be provided with a protective coating to prevent moisture uptake during handling. Such coatings may for example take the form of capsule casings (as described above), tablet coatings, protective film or web coatings, and moisture-proof removable wrappings. Tablet coatings may be applied in conventional manner and may he such as to dissolve in the mouth or stomach (e.g. sugar or sugar/beeswax coatings), or alternatively may be gastric juice resistant polymers (such as the gastric juice resistant Eudragit® coatings produced by Röhm GmbH) where it is desired that drug uptake should occur in the intestines. Protective films or webs may for example be used where the product is to be applied topically, e.g. for uptake across the skin or a toe or finger nail. In this event a pad of the composition will generally be disposed between an adhesive upper protective layer and a lower removable layer. An example of a topical application form for application on nails and adjoining tissue, e.g. for the treatment of fungal infection, is shown in US-A-5181914.

Where the product is produced in particulate form, e.g. by spray-drying, the particles can be loaded into water-tight administration devices (e.g. spray devices or powder dosing devices such as inhalers) for oral, nasal or topical administration of the particulate. Alternatively particulates may be loaded into capsules or mixed with hulking agents such as lactose, starch, microcrystalline cellulose and mixtures thereof, and compressed to form tablets. In any event, the particles may additionally be provided with one or more coatings, e.g. to provide a delayed or prolonged release administration forms.

Generally however it will he preferred to shape the product into individual doses and to provide these with a protective coat, e.g. to produce a capsule, coated tablet or film covered pad single dosage unit.

While not wishing to be bound by theory it is thought that the advantageous drug compound dissolution profile for the compositions of the invention is achieved as a result of a combination of the effects of the components of the composition on exposure to water or aqueous body fluids. The water and the acid provide a highly acidic microenvironment in which the solubility of the basic drug compound is increased. This acidic microenvironment contains the cyclodextrin which is capable of complexing the solubilized drug causing the production of a supersaturated solution of the drug compound and this supersaturated solution is stabilized by the viscosity enhancing effects of the organic polymer which hinders precipitation of the drug as the pH increases as the microenvironment becomes more dilute as more water enters.

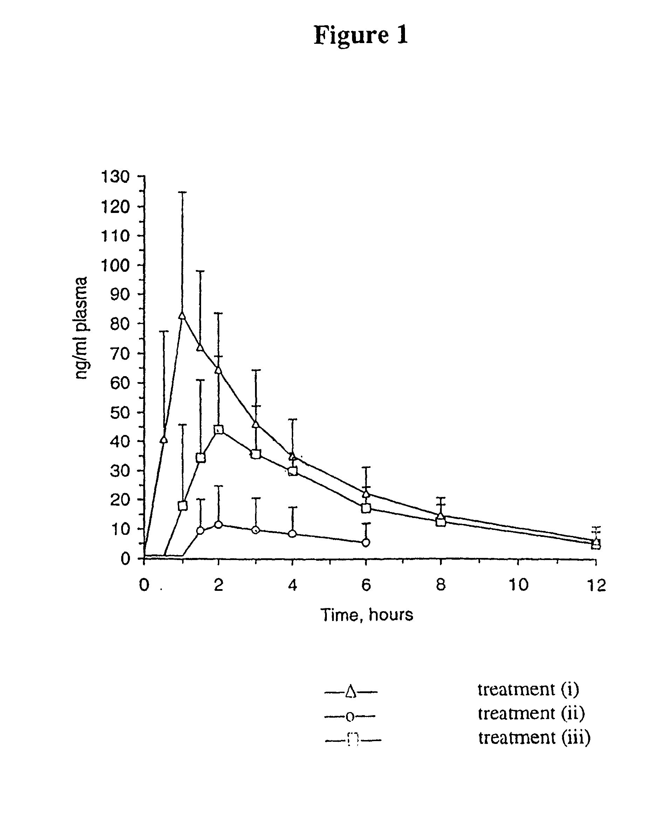

As has been mentioned above, the compositions according to the invention can be produced with particularly favourable drug dissolution profiles. Thus dissolution may be sufficiently rapid to ensure substantially complete availability of the drug compound for biological uptake (e.g. from the mouth, nose, stomach or vagina) yet sufficiently slow to provide a more prolonged plasma uptake profile (see for example Figure 1 of the accompanying drawings) e.g. by avoidance of drug reprecipitation before the composition reaches the stomach.

Such a dissolution profile is thus advantageous in its own right and viewed from a further aspect the pharmaceutical composition according to the invention may comprise at least one water-soluble physiologically tolerable excipient, so that at 5, 15 and 45 minutes after addition of a quantity of said composition containing 100 mg of a basic drug compound to 600 mL of 0.1N hydrochloric acid at 37°C, from 7 to 25 (preferably 10 to 20, especially 12 to 18) %, 45 to 70 (preferably 50 to 65, especially 54 to 63) % and at least 96 (preferably at least 97, especially at least 98) % respectively of said drug compound is in solution in said hydrochloric acid. These figures relate to *in vitro* dissolution studies conducted in accordance with the monograph USP 23, <711> Dissolution, pp. 1791-1793.

For example, in determining the dissolution profiles set out above, the composition is placed without a coating or with a rapidly soluble coating (e.g. a gelatin capsule shell) in 0.1 N HCl (or an other appropriate medium) and the mixture is stirred using the USP-method with a paddle, apparatus 2, at a speed of 50 or 100 rpm.

The compositions according to the invention may be in any form convenient for topical administration or administration into an externally voiding body cavity (e.g. nose, lungs, mouth, ear, stomach, rectum or vagina). Typical administration forms include patches, tablets, buccal tablets, lozenges, can-plugs, nose plugs, coated tablets, capsules, suppositories, chewing gum, gels, powders, granules, syrups and dispersions, although patches and powders and more especially capsules and coated tablets are preferred. The drug dosage will depend upon the drug compound as well as the species and size of the subject being treated. Typically, dosages will be 0.5 to 1.2, preferably 0.8 to 1.05 times the conventional dosages for the selected drug compound administered by the same route.

Further, this invention comprioses a pharmaceutical composition or a pharmaceutical dosage form as described hereinbefore for use in a method of therapy or diagnosis of the human or non-human animal body.

This invention also relates to a pharmaceutical composition for use in the manufacture of a pharmaceutical dosage form for oral administration to a mammal in need of treatment, characterized in that said dosage form can be administered at any time of the day independently of the food taken in by said mammal.

Or, in other words, the present invention also concerns the se of a pharmaceutical composition as described hereinbefore for the manufacture of a pharmaceutical dosage form for oral administration to a mammal in need of treatment, characterized in that said dosage form can be administered at any time of the day independently of the food taken in by said mammal.

This invention also relates to a pharmaceutical package suitable for commercial sale comprising a container, an oral dosage form as claimed in any one of claims 12 to 16, and associated with said package written matter non-limited as to whether the dosage form can be administered with or without food.

The invention will now be described further with reference to the following nonlimiting Examples and the accompanying drawings, in which:
Figures 1 and 2 are graphs showing plasma concentrations of the drug (-)-[2S-[2α,4α(S*)]]-4-[4-[4-[4-[[2-(4-chlorophenyl]-2-[[(4-methyl-4*H*-1,2,4-triazol-3-yl)thio]methyl]-1,3-dioxolan-4-yl]methoxy]phenyl]-1-piperazinyl]phenyl]-2,4-dihydro-2-(1-methylpropyl)-3*H*-1,2,4-triazol-3-one administered in a composition according to the invention and in a conventional administration form (sugar particles coated with the drug and loaded in a gelatin capsule) [see Example 6 for further details]; and
Figure 3 is a dissolution profile for the three itraconazole compositions of Example 2.

### Example 1

### Glass thermoplastic system composition preparation

The following ingredients are mixed in a 250 mL glass flask:

| | |
|---|---|
| Drug compound (e.g. itraconazole) | 20 g |
| Citric acid monohydrate | 100 g |

Anhydrous ethanol (100 mL) is added. The glass flask is placed on a steam bath (bain mane) and stirred at 70°C until the drug and acid are completely dissolved (about 10 minutes). Thereafter the following ingredients are added:

| | |
|---|---|
| Hydroxypropyl-β-cyclodextrin | 50 g |
| Hydroxypropylmethylcellulose (2910.5 mPa.s) | 10 g |

The flask is placed on the steam bath and stirred at 70°C until dissolution is complete (about 70 minutes). The solution is then poured onto cleaned stainless steel plates which are then placed in a drying oven for 2 hours at 80°C under vacuum and subsequently for 40°C under vacuum overnight. The plates are then heated to 80°C and the gel residue is scraped off and filled into 900 mg capacity gelatin capsules (size no. 0).

### Example 2

### Composition preparation

Analogously to Example 1, gelatin capsules having the following relative weights of components are prepared:

| | | |
|---|---|---|
| (A) | 100 mg | Itraconazole |
| | 500 mg | citric acid monohydrate |
| | 275 mg | hydroxypropyl-β-cyclodextrin |
| | 25 mg | Methocel E5 |
| (B) | 100 mg | Itraconazole |
| | 500 mg | citric acid monohydrate |
| | 250 mg | hydroxypropyl-β-cyclodextrin |
| | 50 mg | Methocel E5 |
| (C) | 100 mg | Itraconazole |
| | 500 mg | citric acid monohydrate |
| | 225 mg | hydroxypropyl-β-cyclodextrin |
| | 75 mg | Methocel E5 |
| (D)* | 200 mg | Methyl 6,11-dihydro-11-[1-[2-[4-(2-quinolinylmethoxy)phenyl]ethyl]-4-piperidinylidene]-5*H*-imidazo[2,1-b]-[3]benzazepine 3-carboxylate |
| | 650 mg | citric acid monohydrate |
| | 250 mg | hydroxypropyl-β-cyclodextrin |
| (E) | 100 mg | (-)-[2S-[2α,4α(S*)]]-4-[4-[4-[4-[[2-(4-chlorophenyl]-2-[[(4-methyl-4*H*-1,2,4-triazol-3-yl)thio[methyl]-1,3-dioxolan-4-yl]methoxy]-phenyl]-1-piperazinyl]phenyl]-2,4-dihydro-2-(1-methylpropyl)-3*H*-1,2,4-triazol-3-one |
| | 500 mg | citric acid monohydrate |
| | 250 mg | hydroxypropyl-β-cyclodextrin |
| | 50 mg | Methocel E5 |

| | | |
|---|---|---|
| * For example 2(D) the composition is loaded into 1100 mg gelatin capsules. | | |

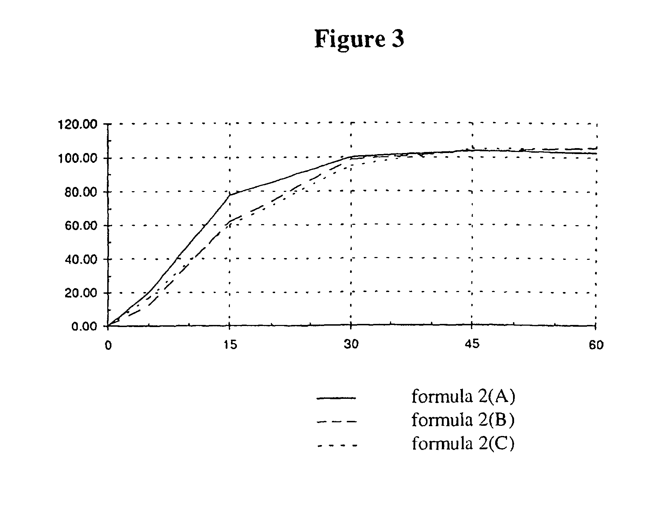

The dissolution profiles of the gels of Example 2(A), (B) and (C) are shown in Figure 3 of the accompanying drawings. These were determined by placing one capsule containing 100 mg of itraconazole in 300 mL of stirred 0.1 N HCl at 37°C and observing the percentage of dissolved drug compound at times 0, 5, 15, 30, 45 and 60 minutes (stirring was effected using the USP-method with paddle, apparatus 2, 100 rpm). For Example 2(E), with 100 mg drug compound added to 600 mL of 0.1 N HCl at 37°C, the mean percentages of drug compound in solution at 5, 15, 30 and 45 minutes were 17.22, 61.18, 92.73 and 98.67 respectively (stirring was effected using the USP-method with paddle, apparatus 2, 100 rpm).

The dissolution profile of Example 2(E) was compared with that of a conventional capsule dosage form in which the gelatin capsule is loaded with sugar particles coated with 100 mg of (-)-[2S-[2α,4α(S*)]]-4-[4-[4-[4-[[2-(4-chlorophenyl]-2-[[(4-methyl-4*H*-1,2,4-triazol-3-yl)thio]methyl]-1,3-dioxolan-4-yl]methoxy]phenyl]-1-piperazinyl]-phenyl]-2,4-dihydro-2-(1-methylpropyl)-3*H*-1,2,4-triazol-3-one. The capsules were placed in 10 ml of 0.1 N HCl at 37°C in glass vials and shaken in a mechanical shaker (100 strokes per minute) and the percentage of drug compound in solution after 0, 30 and 60 minutes was determined. The results are set out in Table 1 below.

**Table 1**

| Percentage of drug compound in solution | | |
|---|---|---|
| Time | Example 2(E) | Conventional Capsule |
| 0 | 0 | 0 |
| 30 | 91.26 | 15.54 |
| 60 | 101.90 | 18.39 |

This clearly shows how much more readily the drug compound is made bioavailable by the compositions of the invention.

### Example 3

### Effect of organic polymer on supersaturation stability

Aqueous solutions of hydroxypropyl-β-cyclodextrin (HPβCD) anti Methocel E5 in 300 ml 0.1 N HCl at 37"C were prepared having the concentrations set out in Table 2. The solutions were stirred using the USP-method with paddle, apparatus 2, 150 rpm.

| Sample | HPβCD (mg) | Methocel E5 (mg) |
|---|---|---|
| 1 | 250 | 250 |
| 2 | 500 | 0 |
| 3 | 250 | 00 |
| 4 | 500 | 500 |
| 5 | 0 | 250 |
| 6 | 500 | 250 |
| 7 | 0 | 0 |
| 8 | 250 | 0 |
| 9 | 0 | 500 |

To these solutions, with stirring, a concentrated solution of itraconazole in DMF (50 mg/mL) was added dropwise until precipitation was observed. Subsequently the concentration of dissolved itraconazole expressed in mg% (ie. the number of mg dissolved in 100 mL) was observed at 0, 30, 60 and 120 minutes. The results are set out in Table 3 below:

**Table 3**

| Percentage of drug compound in solution | | | | | | | | | |
|---|---|---|---|---|---|---|---|---|---|
| Sample Time (minutes) | 1 | 2 | 3 | 4 | 5 | 6 | 7 | 8 | 9 |
| 0 | 59.52 | 72.10 | 58.95 | 75.47 | 42.65 | 75.27 | 42.60 | 60.95 | 42.95 |
| 30 | 62.02 | 74.40 | 62.05 | 78.12 | 44.85 | 80.17 | 44.10 | 62.92 | 45.20 |
| 60 | 62.52 | 70.37 | 62.50 | 79.47 | 45.40 | 80.40 | 44.97 | 64.07 | 46.00 |
| 120 | 62.79 | 45.82 | 63.90 | 80.77 | 46.55 | 81.25 | 31.32 | 33.65 | 47.05 |
| HPBCD: | 1:1 | 2:0 | 1:2 | 2:2 | 0:1 | 2:1 | 0:0 | 1:0 | 0:2 |
| Methocel ratio | | | | | | | | | |

These results clearly demonstrate (i) the solubilizing effect of the cyclodextrin (Samples 2, 6 and 4 show the highest initial itraconazole concentrations, followed by Samples 8, 1 and 3, with Samples 7, 5 and 9 showing the lowest initial concentrations) and (ii) the stabilizing effect of the organic polymer (Samples 2, 8 and 7 show the greatest drop in itraconazole concentrations over 120 minutes, etc).

### Example 4

### Extended release formulation

Analogously to Example 1, gelatin capsules were prepared containing the following:

| | |
|---|---|
| 41.55 mg | Cisapride |
| 508.45 mg | citric acid monohydrate |
| 250 mg | hydroxypropyl-β-cyclodextrin |
| 100 mg | Methocel K15M |

This formulation has a much slower dissolution rate than the compositions of Example 2. However the rate of dissolution is much more close to linear with time and shows much less dependence on the pH of the dissolution medium.

### Example 5

### Nail gel

A gel for application to nails or hooves to effect antifungal treatment is made with the following composition:

| | |
|---|---|
| Itraconazole | 250 mg |
| Citric acid monohydrate | 2083 mg |
| Hydroxypropyl-β-cyclodextrin | 333 mg |
| Hydroxypropylmethylcellulose (Methocel E5) | 83 mg |
| Anhydrous ethanol | 2 ml |

### Example 6

### Body uptake

The plasma concentrations of R 103757 were determined in healthy humans at 0, ½, 1, 1½, 2, 3, 4, 6, 8 and 12 hours after oral administration of 100 mg (-)-[2S-[2α,4α(S*)]]-4-[4-[4-[4-[[2-(4-chlorophenyl]-2-[[(4-methyl-4*H*-1,2,4-triazol-3-yl)-thio]methyl]-1,3-dioxolan-4-yl]methoxy]phenyl]-1-piperazinyl]phenyl]-2,4-dihydro-2-(1-methylpropyl)-3*H*-1,2,4-triazol-3-one as (i) a 5 mg/mL oral solution containing 25% hydroxypropyl-β-cyclodextrin solution administered under fasting conditions, (ii) a conventional capsule containing (-)-[2S-[2α,4α(S*)]]-4-[4-[4-[4-[[2-(4-chlorophenyl]-2-[[(4-methyl-4*H*-1,2,4-triazol-3-yl)thio]methyl]-1,3-dioxolan-4-yl]methoxy]phenyl]-1-piperazinyl]phenyl]-2,4-dihydro-2-(1-methylpropyl)-3*H*-1,2,4-triazol-3-one coated onto sugar particles administered under fasting conditions, (iii) a conventional capsule containing (-)-[2S-[2α,4α(S*)]]-4-[4-[4-[4-[[2-(4-chlorophenyl]-2-[[(4-methyl-4*H*-1,2,4-triazol-3-yl)thio]methyl]-1,3-dioxolan-4-yl]methoxy]phenyl]-1-piperazinyl]-phenyl]-2,4-dihydro-2-(1-methylpropyl)-3*H*-1,2,4-triazol-3-one coated onto sugar particles administered after a standard breakfast, (iv) a capsule according to Example 2(E) administered under fasting conditions and (v) a capsule according to Example 2(E) administered after a standard breakfast.

The "standard breakfast" comprised four slices of bread, one slice of ham, one slice of cheese, butter, jelly and two cups of coffee or tea with milk and/or sugar if desired. The 100 mg dose of (-)-[2S-[2α,4α(S*)]]-4-[4-[4-[4-[[2-(4-chlorophenyl]-2-[[(4-methyl-4*H*-1,2,4-triazol-3-yl)thio]methyl]-1,3-dioxolan-4-yl]methoxy]phenyl]-1-piperazinyl]phenyl]-2,4-dihydro-2-(1-methylpropyl)-3*H*-1,2,4-triazol-3-one was administered just after such a breakfast.

Blood samples of 10 mL were taken to obtain 5 mL plasma. The blood samples were taken, collected in heparinized tubes, and centrifuged at 1000g for 10 minutes within 2 hours of collection. Plasma was transferred into plastic tubes, which were sealed and stored at -70°C until assayed.

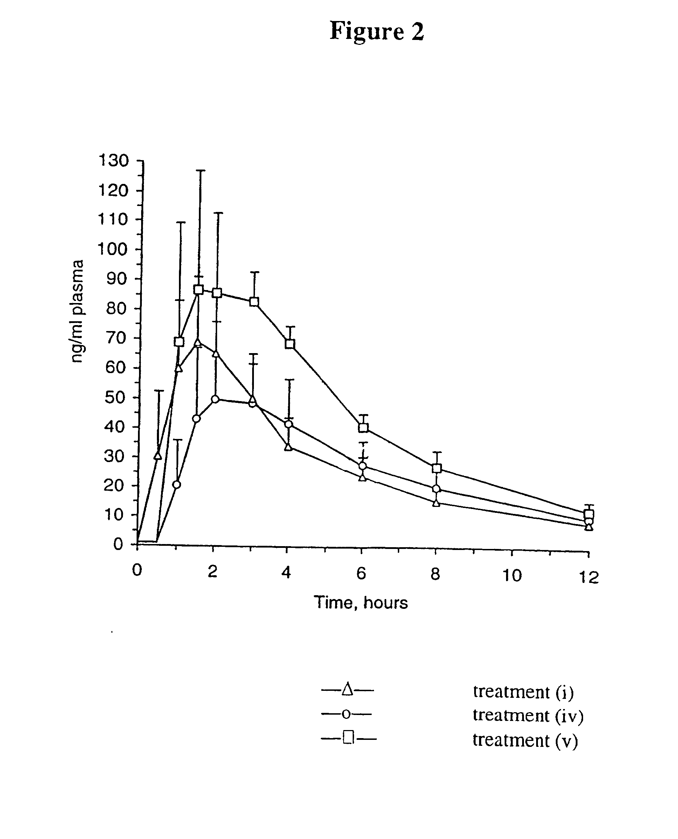

The results are shown in Figures 1 and 2 which presents drug concentrations as a function of time. As can be seen, the conventional capsule performs significantly worse than the solution even with fasting. However the capsule according to the invention outperforms the solution after 3 hours whether or not the recipient has fasted and, most surprisingly, completely outperforms the solution where the recipient has not fasted.

### Example 7

### Effect of pH on dissolution rate

Following the procedure of Example 1, a placebo capsule comprising methylene blue (2,63 mg), citric acid (600 mg), hydroxypropyl-β-cyclodextrin (250 mg) and hydroxypropylmethylcellulose (Methocel E5, 50 mg) was prepared. The dissolution of these capsules was determinated at various pH values according to the USP method (600 ml medium, 37°C, Apparatus 2 with paddle, 100 rpm). The six media tested were : 0.1N HCl (pH 1.55), 0.01N HCl (pH 2.25), 0.001N HCl (pH 2.75), USP pH 4.5 (pH 4.40), USP pH 6.5 (pH 5.80) and USP pH 7.5 (pH 7.0).

The results are set out in table 4 below :

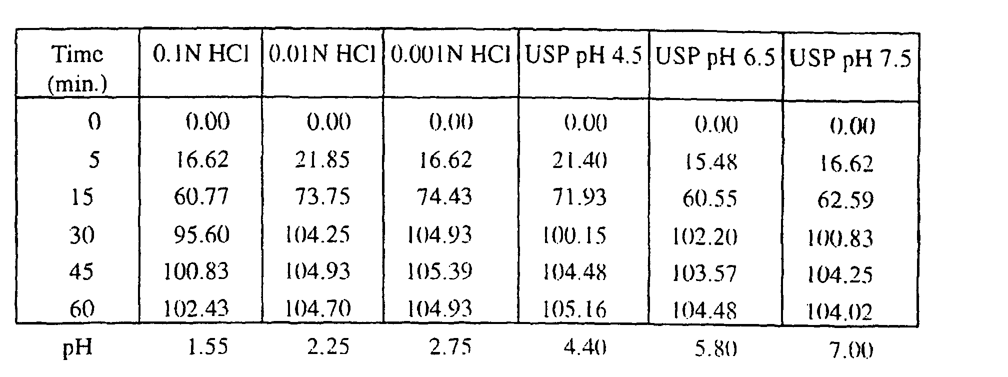

### Example 8

Following the procedure of Example 1, various drug containing capsules were made having the following relative weights of components :

| | | |
|---|---|---|
| A. | 100 mg | itraconazole |
| | 500 mg | citric acid |
| | 250 mg | hydroxypropyl-β-cyclodextrin |
| | 50 mg | HPMC E5 |
| B. | 200 mg | methyl 6,11-dihydro-11-[1-[2-[4-(2-quinolinylmethoxy)phenyl]ethyl]-4-piperidinylidene]-5*H*-imidazo[2,1-b][3]benzazepine-3-carboxylate |
| | 650 mg | citric acid |
| | 250 mg | hydroxypropyl-β-cyclodextrin |
| C. | 100 mg | (-)-[2S-[2α,4α(S*)]]-4-[4-[4-[4-[[2-(4-methyl-4*H*-1,2,4-triazol-3-yl)thio]methyl]-1,3-dioxolan-4-yl]methoxy]phenyl]-1-piperazinyl]phenyl]-2,4-dihydro-2-(1-methylpropyl)-3*H*-1,2,4-triazol-3-one |
| | 500 mg | citric acid |
| | 250 mg | hydroxypropyl-β-cyclodextrin |
| | 50 mg | HPMC E5 |
| D. | 100 mg | 4-[[4-amino-6-[(2,6-dichlorophenyl)methyl]-1,3,5-triazin-2-yl]amino]-benzonitrile |
| | 500 mg | citric acid |
| | 250 mg | hydroxypropyl-β-cyclodextrin |
| | 50 mg | HPMC E5 |
| E. | 5 mg | (B)-N-[4-[2-ethyl-1-(1H-1,2,4-triazol-1-yl)butyl]phenyl]-2-benzo- |
| | | thiazolamine |
| | 500 mg | citric acid |
| | 395 mg | hydroxypropyl-β-cyclodextrin |
| F. | 100 mg | (B-cis)-1-[4-[4-[4-[[4-(2,4-difluorophenyl)-4-(1*H*-1,2,4-triazol-1-yl-methyl)-1,3-dioxolan-2-yl]methoxy]phenyl]-1-piperazinyl]phenyl]-3-(1-methylethyl)-2-imidazolidinone |
| | 500 mg | citric acid |
| | 250 mg | hydroxypropyl-β-cyclodextrin |
| | 50 mg | HPMC E5 |

The dissolution of these compositions was determined according to the USP method (600 ml 0.1 N HCl, 37°C, Apparatus 2 with paddle, 100 ppm), except formulation (A) where only 300 ml medium was used. The results are set out in the following tables 5-10 :

**Table 5 :**

| Formulation (A) | | | |
|---|---|---|---|
| Time (min) | sample 1 | sample 2 | sample 3 |
| 0 | 0.00 | 0.00 | 0.00 |
| 5 | 10.75 | 9.99 | 10.69 |
| 15 | 56.61 | 57.18 | 59.61 |
| 30 | 85.89 | 88.98 | 90.24 |
| 45 | 95.46 | 99.84 | 96.87 |
| 60 | 101.94 | 102.06 | 102.87 |

**Table 6 :**

| Formulation (B) | | | |
|---|---|---|---|
| Time (min) | 0.1N HCl | 0.01N HCl | 0.001N HCl |
| 0 | 0.00 | 0.00 | 0.00 |
| 5 | 27.00 | 25.17 | 21.39 |
| 15 | 92.13 | 86.94 | 84.75 |
| 30 | 97.11 | 96.63 | 93.09 |
| 45 | 98.64 | 99.45 | 94.83 |
| 60 | 100.29 | 100.08 | 95.28 |

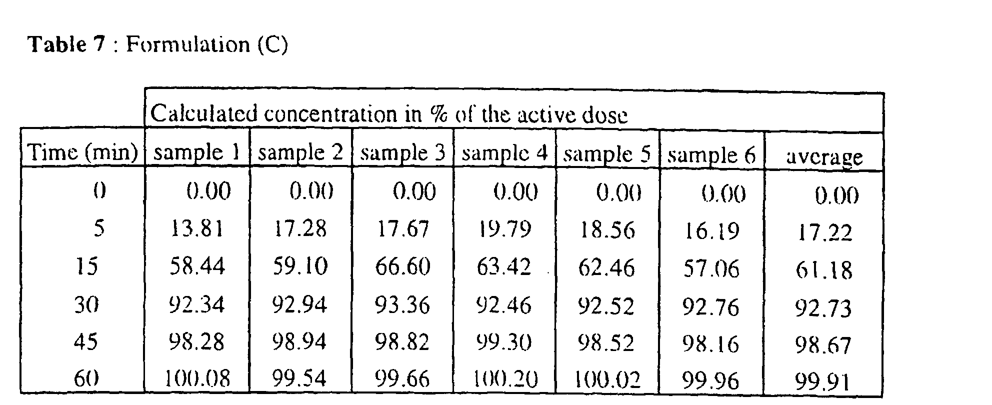

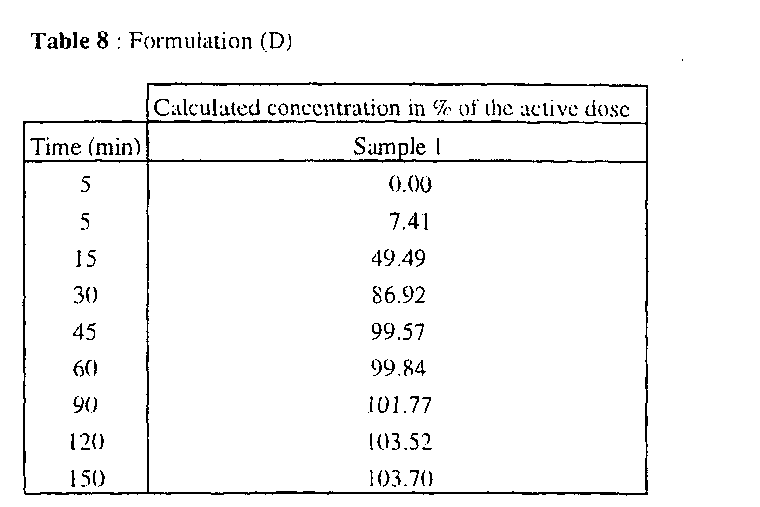

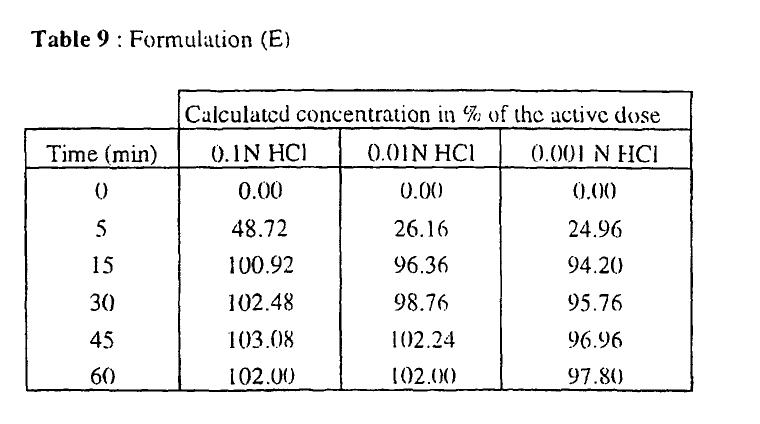

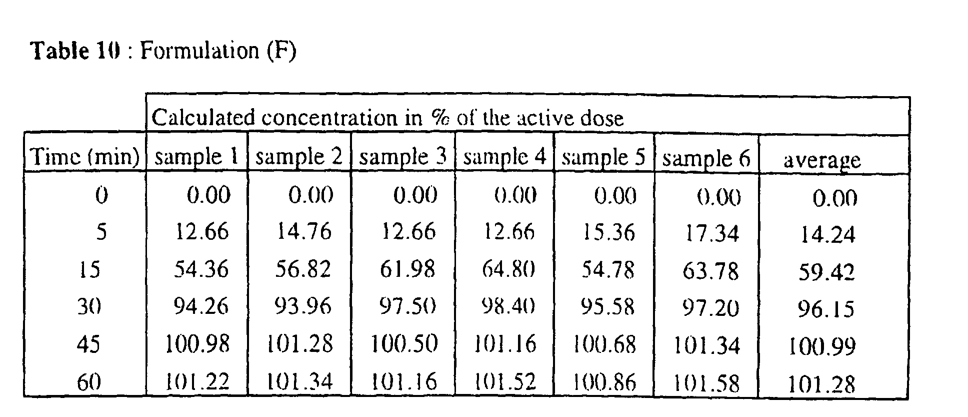

### Example 9

### Stability testing of formulation 8 (C)

Capsules of formulation 8(C) were stored for 1 month and 3 months at 40°C, and for 1 year at room temperature. Dissolution measurements were made according to the USP method (600 ml 0.1N HCl, 37°C, paddle apparatus 2.100 rpm). The following results were obtained :

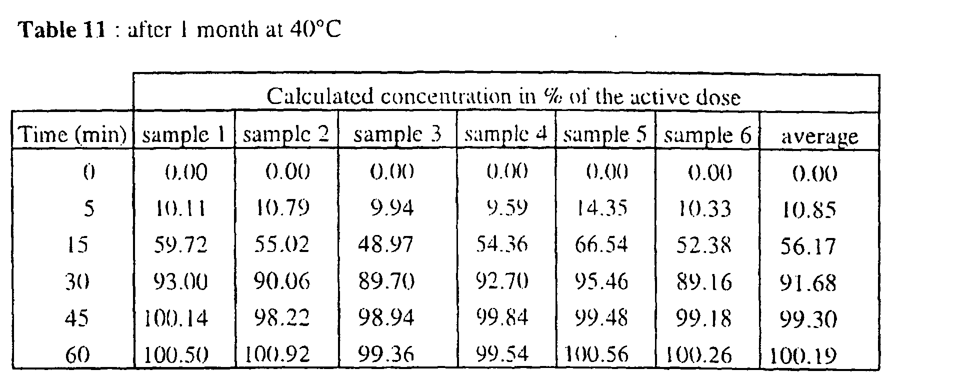

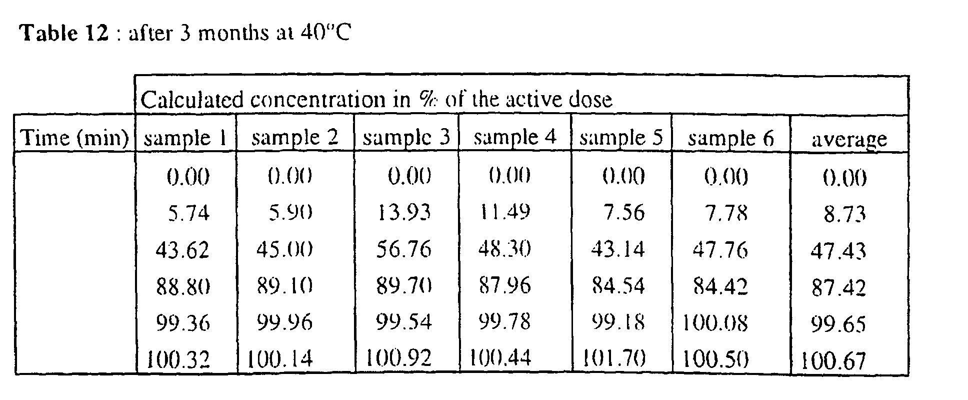

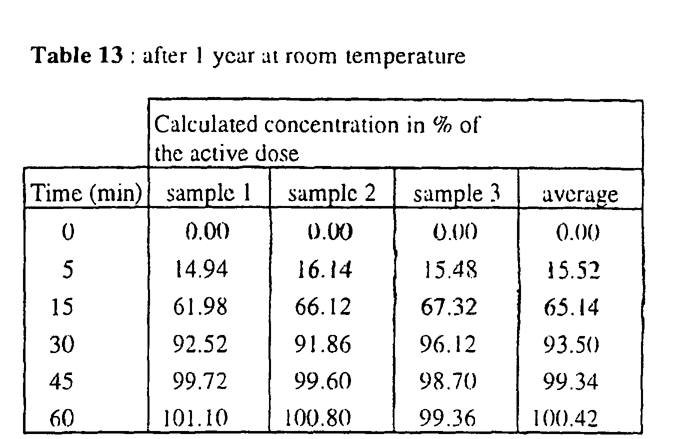

### Example 10

### Variability in bioavailability of Formulation (D)

The variability in the bioavailability of Formulation (D) in beagle dogs was evaluated as follows. First, two beagle dogs received as single oral administration of a PEG-400 solution comprising 4-[[4-amino-6-[(2,6-dichlorophenyl)methyl]-1,3,5-triazin-2-yl]amino]benzonitrile at a dose of 10 mg/kg. Plasm levels were measured for 32 hours. After 7 days, the same dogs were now treated with a single oral capsule comprising the formula (D) at 10 mg/kg. Plasm levels were again determined for up to 32 hours after administration. The individual results are as follows.

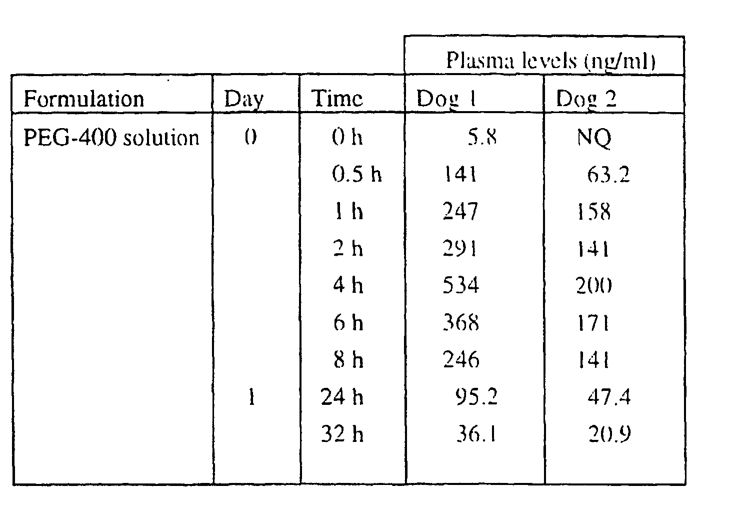

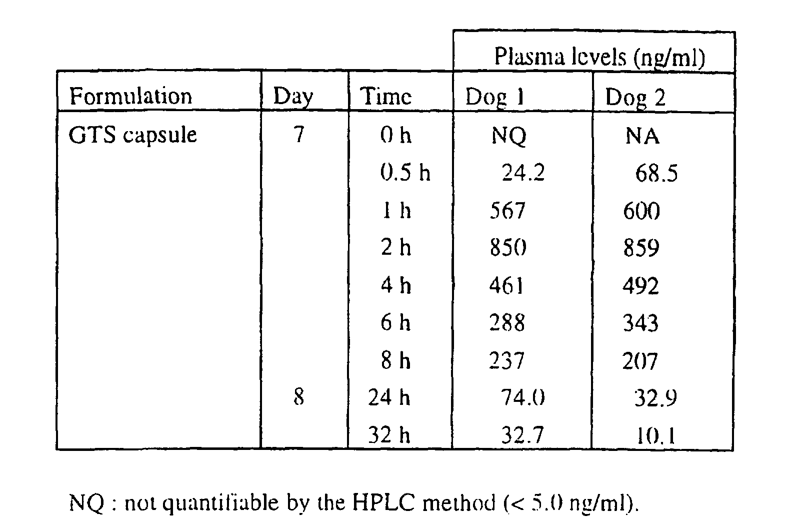

Surprisingly, the plasm levels obtained after administration of the capsules comprising formula (D) are very much more similar to one another in the two test animals than those obtained after administration of the PEG 400 solution.

### Example 11

### Permeation and accumulation of itraconazole through and in human skin

A Franz cell was fitted with fresh whole human skin and its receptor filled with a 20% (w/v) solution of hydroxypropyl-β-cyclodextrin in water. A Finn Chambers patch was filled with Formulation 8(A) and was then placed on the skin wetted with a small amount of phosphate buffered saline. Samples of the receptor solution were withdrawn at regular intervals and the presence of itraconazole in the solution was measured using high performance liquid chromatography. At no time point could any trace of itraconazole be detected, indicating that this compound did not penetrate whole human skin. At the end of the experiment the skin was thoroughly washed and then extracted in order to determine the amount of itraconazole accumulated in the skin. A mean value of 12.2 µg/cm² could be calculated from the results of 8 independent experiments.

## Claims

1. A pharmaceutical composition comprising a no more than sparingly water-soluble basic drug compound, a cyclodextrin, a physiologically tolerable water-soluble acid, and a physiologically tolerable water-soluble organic polymer, **characterized in that** it comprises from 35 to 95 % by weight of acid relative to the total weight of cyclodextrin, drug compound, organic polymer and acid.

2. The composition of claim 1 **characterised in that** the weight ratios of drug compound to acid and of drug compound to cyclodextrin are no more than 2:1.

3. The composition of claim 1 or 2 **characterized in that** the components of the composition are so dispersed as to form a chemically and physically uniform or homogeneous system or to consist of one phase.

4. The composition of claim 3 wherein the cyclodextrin is 2-hydroxypropyl-β-cyclodextrin.

5. The composition of claim 3 wherein the acid is selected from the group comprising citric, fumaric, tartaric, maleic, malic, succinic, oxalic, malonic, benzoic, mandelic and ascorbic acid.

6. The composition of claim 5 wherein the acid is citric acid.

7. The composition of claim 3 wherein the polymer is selected from the group comprising
- alkylcelluloses such as methylcellulose,
- hydroxyakylcelluloses such as hydroxymethylcellulose, hydroxyethylcellulose, hydroxypropylcellulose and hydroxybutylcellulose,
- hydroxyalkyl alkylcelluloses such as hydroxyethyl methylcellulose and hydroxypropyl methylcellulose,
- carboxyalkylcelluloses such as carboxymethylcellulose,
- alkali metal salts of carboxyalkylcelluloses such as sodium carboxymethylcellulose,
- carboxyalkylalkylcelluloses such as carboxymethylethylcellulose,
- carboxyalkylcellulose esters,
- starches,
- pectins such as sodium carboxymethylamylopectin,
- chitin derivates such as chitosan,
- heparin and heparinoids,
- polysaccharides such as alginic acid, alkali metal and ammonium salts thereof, carrageenans, galactomannans, tragacanth, agar-agar, gum arabic, guargum and xanthan gum,
- polyacrylic acids and the salts thereof,
- polymethacrylic acids and the salts thereof, methacrylate copolymers,
- polyvinylalcohol,
- polyvinylpyrrolidone, copolymers of polyvinylpyrrolidone with vinyl acetate,
- polyalkylene oxides such as polyethylene oxide and polypropylene oxide and copolymers of ethylene oxide and propylene oxide, e.g. poloxamers and poloxamines.

8. The composition of claim 7 wherein the polymer has an apparent viscosity of 1 - 100 mPa.s when dissolved in a 2% aqueous solution at 20°C.

9. The composition of claim 8 wherein the polymer is hydroxypropylmethylcellulose.

10. A composition according to any one of the preceding claims that dissolves rapidly in body fluids, **characterized in that** it comprises from 50 to 95 % by weight of acid.

11. A composition according to any one of the preceding claims that provides sustained release of the drug, **characterized in that** it comprises a water soluble polymer having an apparent viscosity of more than 1,000 mPa.s when dissolved in a 2% aqueous solution at 20°C.

12. A pharmaceutical dosage form comprising a therapeutically effective amount of a pharmaceutical composition as defined in any one of the preceding claims.

13. The dosage form of claim 12 adapted for topical administration or administration into an externally voiding body cavity such as the nose, lungs, mouth, ear, stomach, rectum and vagina.

14. The dosage form of claim 12 wherein said composition is filled into a standard capsule, or alternatively is mixed with bulking agents and compressed into tablets.

15. The dosage form of claim 12, **characterised in that** at 5, 15 and 45 minutes after addition of said dosage form to 0.1N hydrochloric acid at 37°C in the dissolution test set forth in USP test <711> in a USP-2 dissolution apparatus equiped with a paddle, from 7 to 25%, 45 to 70% and at least 96% respectively of drug is dissolved in said 0.1 N hydrochloric acid.

16. A pharmaceutical composition according to any one of claims 1 to 11 or a pharmaceutical dosage form according to any one of claims 12 to 15 for use in a method of therapy or diagnosis of the human or non-human animal body.

17. A pharmaceutical composition according to any one of claims 1 to 11 for use in the manufacture of a pharmaceutical dosage form for oral administration to a mammal in need of treatment, **characterized in that** said dosage form can be administered at any time of the day independently of the food taken in by said mammal.

18. Use of a pharmaceutical composition according to any one of claims 1 to 11 for the manufacture of a pharmaceutical dosage form for oral administration to a mammal in need of treatment, **characterized in that** said dosage form can be administered at any time of the day independently of the food taken in by said mammal.

19. Use of pharmaceutical composition according to any one of claims 1 to 11 for the manufacture of a medicament for use in a method of therapy or diagnosis of the human or non-human body.

20. A pharmaceutical package suitable for commercial sale comprising a container, an oral dosage form as claimed in any one of claims 12 to 16, and associated with said package written matter non-limited as to whether the dosage form can be administered with or without food.

## Patentansprüche

1. Pharmazeutische Zusammensetzung, enthaltend eine höchstens geringfügig wasserlösliche, basische Arzneimittelverbindung, ein Cyclodextrin, eine physiologisch unbedenkliche wasserlösliche Säure und ein physiologisch unbedenkliches wasserlösliches organisches Polymer, **dadurch gekennzeichnet, daß** sie von 35 bis 95 Gew.-% Säure, bezogen auf das Gesamtgewicht an Cyclodextrin, Arzneimittelverbindung, organischem Polymer und Säure, enthält.

2. Zusammensetzung nach Anspruch 1, **dadurch gekennzeichnet, daß** das Gewichtsverhältnis von Arzneimittelverbindung zu Säure und von Arzneimittelverbindung zu Cyclodextrin nicht mehr als 2:1 beträgt.

3. Zusammensetzung nach Anspruch 1 oder 2, **dadurch gekennzeichnet, daß** die Komponenten der Zusammensetzung so dispergiert sind, daß sie ein chemisch und physikalisch einheitliches oder homogenes System bilden oder aus einer Phase bestehen.

4. Zusammensetzung nach Anspruch 3, wobei es sich bei dem Cyclodextrin um 2-Hydroxypropyl-β-cyclodextrin handelt.

5. Zusammensetzung nach Anspruch 3, wobei die Säure aus der aus Citronensäure, Fumarsäure, Weinsäure, Maleinsäure, Äpfelsäure, Bernsteinsäure, Oxalsäure, Benzoesäure, Mandelsäure und Ascorbinsäure bestehenden Gruppe ausgewählt ist.

6. Zusammensetzung nach Anspruch 5, wobei es sich bei der Säure um Citronensäure handelt.

7. Zusammensetzung nach Anspruch 3, wobei das Polymer aus der aus
- Alkylcellulosen wie Methylcellulose,
- Hydroxyalkylcellulosen wie Hydroxymethylcellulose, Hydroxyethylcellulose, Hydroxypropylcellulose und Hydroxybutylcellulose,
- Hydroxyalkylalkylcellulosen wie Hydroxyethylmethylcellulose und Hydroxypropylmethylcellulose,
- Carboxyalkylcellulosen wie Carboxymethylcellulose,
- Alkalisalzen von Carboxyalkylcellulosen wie Natriumcarboxymethylcellulose,
- Carboxyalkylalkylcellulosen wie Carboxymethylethylcellulose,
- Carboxyalkylcelluloseestern,
- Stärken,
- Pektinen wie Natriumcarboxymethylamylopektin,
- Chitinderivaten wie Chitosan,
- Heparin und Heparinoiden,
- Polysacchariden wie Alginsäure und deren Alkali- und Ammoniumsalze, Carrageenanen, Galactomannanen, Tragacanthgummi, Agar-Agar, Gummi arabicum, Guargummi und Xanthangummi,
- Polyacrylsäuren und deren Salzen,
- Polymethacrylsäuren und deren Salzen, Methacrylatcopolymeren,
- Polyvinylalkohol,
- Polyvinylpyrrolidon, Copolymeren von Polyvinylpyrrolidon mit Vinylacetat,
- Polyalkylenoxiden wie Polyethylenoxid und Polypropylenoxid und Copolymeren von Ethylenoxid und Propylenoxid, z.B. Poloxameren und Poloxaminen,
bestehenden Gruppe ausgewählt ist.

8. Zusammensetzung nach Anspruch 7, wobei das Polymer eine scheinbare Viskosität von 1 - 100 mPa.s aufweist, wenn es bei 20°C in einer 2%igen wäßrigen Lösung gelöst wird.

9. Zusammensetzung nach Anspruch 8, wobei es sich bei dem Polymer um Hydroxypropylmethylcellulose handelt.

10. Zusammensetzung nach einem der vorhergehenden Ansprüche, die sich schnell in Körperflüssigkeiten löst, **dadurch gekennzeichnet, daß** sie von 50 bis 95 Gew.-% Säure enthält.

11. Zusammensetzung nach einem der vorhergehenden Ansprüche, die für eine retardierte Freisetzung des Arzneimittels sorgt, **dadurch gekennzeichnet, daß** sie ein wasserlösliches Polymer enthält, das, wenn es bei 20°C in einer 2%igen wäßrigen Lösung gelöst wird, eine scheinbare Viskosität von über 1000 mPa.s aufweist.

12. Pharmazeutische Dosierungsform, enthaltend eine therapeutisch wirksame Menge einer nach einem der vorhergehenden Ansprüche definierten pharmazeutischen Zusammensetzung.

13. Dosierungsform nach Anspruch 12, die für eine topische Verabreichung oder eine Verabreichung in eine mit der Umgebung in Kontakt stehende Körperhöhle wie der Nase, den Lungen, dem Mund, dem Ohr, dem Magen, dem Rektum und der Vagina ausgelegt ist.

14. Dosierungsform nach Anspruch 12, wobei die Zusammensetzung in eine Standardkapsel abgefüllt wird oder alternativ mit Füllstoffen gemischt und zu Tabletten verpreßt wird.

15. Dosierungsform nach Anspruch 12, **dadurch gekennzeichnet, daß** 5, 15 und 45 Minuten nach Zugabe der Dosierungsform zu 0,1 N Salzsäure bei 37°C in dem im USP-Test <711> beschriebenen Auflösungstest in einem mit einem Rührer ausgestatteten USP-2-Auflösungsapparat von 7 bis 25%, von 45 bis 70% bzw. wenigstens 96% des Arzneimittels in der 0,1 N Salzsäure gelöst ist.

16. Pharmazeutische Zusammensetzung nach einem der Ansprüche 1 bis 11 oder pharmazeutische Dosierungsform nach einem der Ansprüche 12 bis 15 zur Verwendung in einem Therapie- oder Diagnoseverfahren am menschlichen Körper oder nichtmenschlichen Tierkörper.

17. Pharmazeutische Zusammensetzung nach einem der Ansprüche 1 bis 11 zur Verwendung bei der Herstellung einer pharmazeutischen Dosierungsform zur oralen Verabreichung an ein einer Behandlung bedürftiges Säugetier, **dadurch gekennzeichnet, daß** die Dosierungsform dem Säugetier zu jeder Tageszeit unabhängig von der Nahrungsmittelaufnahme verabreicht werden kann.

18. Verwendung einer pharmazeutischen Zusammensetzung nach einem der Ansprüche 1 bis 11 zur Herstellung einer pharmazeutischen Dosierungsform zur oralen Verabreichung an ein einer Behandlung bedürftiges Säugetier, **dadurch gekennzeichnet, daß** die Dosierungsform dem Säugetier zu jeder Tageszeit unabhängig von der Nahrungsmittelaufnahme verabreicht werden kann.

19. Verwendung einer pharmazeutischen Zusammensetzung nach einem der Ansprüche 1 bis 11 zur Herstellung eines Medikaments zur Verwendung in einem Therapie- oder Diagnoseverfahren am menschlichen oder nichtmenschlichen Körper.

20. Pharmazeutische Packung, geeignet für den kommerziellen Verkauf, enthaltend ein Behältnis, eine orale Dosierungsform nach einem der Ansprüche 12 bis 16 und eine gedruckte Packungsbeilage, wobei nicht eingeschränkt wird, ob die Dosierungsform mit oder ohne Nahrungsmittel verabreicht werden kann.

## Revendications

1. Composition pharmaceutique comprenant un composé médicinal basique pas plus que peu hydrosoluble, une cyclodextrine, un acide hydrosoluble physiologiquement tolérable, et un polymère organique hydrosoluble physiologiquement tolérable, **caractérisée en ce qu'**elle comprend de 35 à 95 % en poids d'acide par rapport au poids total de cyclodextrine, de composé médicinal, de polymère organique et d'acide.

2. Composition selon la revendication 1, **caractérisée en ce que** les rapports en poids du composé médicinal à l'acide et du composé médicinal à la cyclodextrine ne sont pas plus que 2:1.

3. Composition selon la revendication 1 ou 2,
**caractérisée en ce que** les composants de la composition sont dispersés de manière à former un système chimiquement et physiquement uniforme ou homogène ou à constituer une phase.

4. Composition selon la revendication 3, **caractérisée en ce que** la cyclodextrine est la 2-hydroxypropyl-β-cyclodextrine.

5. Composition selon la revendication 3, **caractérisée en ce que** l'acide est choisi dans le groupe comprenant l'acide citrique, fumarique, tartrique, maléique, malique, succinique, oxalique, malonique, benzoïque, mandélique et ascorbique.

6. Composition selon la revendication 5, **caractérisée en ce que** l'acide est l'acide citrique.

7. Composition selon la revendication 3, **caractérisée en ce que** le polymère est choisi dans le groupe comprenant
■ les alkylcelluloses telles que la méthylcellulose,
■ les hydroxyalkylcelluloses telles que l'hydroxyméthylcellulose, l'hydroxyéthylcellulose, l'hydroxypropylcellulose et l'hydroxybutyl-cellulose,
■ les hydroxyalkylalkylcelluloses telles que l'hydroxyéthylméthylcellulose et l'hydroxypropyl-méthylcellulose,
■ les carboxyalkylcelluloses telles que la carboxyméthylcellulose,
■ les sels alcalins des carboxyalkylcelluloses telles que la carboxyméthylcellulose sodique,
■ les carboxyalkylalkylcelluloses telles que la carboxyméthyléthylcellulose,
■ les esters de carboxyalkylcellulose,
■ les amidons,
■ les pectines telles que la carboxyméthylamylo-pectine sodique,
■ les dérivés de chitine tels que le chitosane,
■ l'héparine et les héparinoïdes,
■ les polysaccharides tels que l'acide alginique, les sels alcalins et d'ammonium de ceux-ci, les carraghénanes, les galactomannanes, la gomme tragacanthe, la gélose, la gomme arabique, la gomme guar et la gomme xanthane,
■ les acides polyacryliques et leurs sels,
■ les acides polyméthacryliques et leurs sels, les copolymères méthacrylates,
■ l'alcool polyvinylique,
■ la polyvinylpyrrolidone, les copolymères de polyvinylpyrrolidone avec l'acétate de vinyle,
■ les polyoxydes d'alkylène tels que le polyoxyde d'éthylène et le polyoxyde de propylène et les copolymères d'oxyde d'éthylène et d'oxyde de propylène, par ex. les poloxamères et les poloxamines.

8. Composition selon la revendication 7, **caractérisée en ce que** le polymère a une viscosité apparente de 1 - 100 mPa.s lorsqu'il est dissous dans une solution aqueuse à 2 % à 20°C.

9. Composition selon la revendication 8, **caractérisée en ce que** le polymère est l'hydroxypropyl-méthylcellulose.

10. Composition selon l'une quelconque des revendications précédentes qui se dissout rapidement dans les liquides corporels, **caractérisée en ce qu'**elle comprend de 50 à 95 % en poids d'acide.

11. Composition selon l'une quelconque des revendications précédentes qui donne une libération prolongée du médicament, **caractérisée en ce qu'**elle comprend un polymère hydrosoluble ayant une viscosité apparente supérieure à 1 000 mPa.s lorsqu'il est dissous dans une solution aqueuse à 2 % à 20°C.

12. Forme pharmaceutique comprenant une quantité thérapeutiquement efficace d'une composition pharmaceutique telle que définie dans l'une quelconque des revendications précédentes.

13. Forme pharmaceutique selon la revendication 12, adaptée à l'administration par voie topique ou à l'administration dans une cavité corporelle à évacuation externe telle que le nez, les poumons, la bouche, l'oreille, l'estomac, le rectum et le vagin.

14. Forme pharmaceutique selon la revendication 12, **caractérisée en ce que** ladite composition est mise dans une capsule standard, ou de manière alternative est mélangée avec des agents de masse et mise en comprimés.

15. Forme pharmaceutique selon la revendication 12, **caractérisée en ce qu'**à 5, 15 et 45 minutes après l'addition de ladite forme pharmaceutique à de l'acide chlorhydrique 0,1 N à 37°C dans le test de dissolution énoncé dans le test USP <711> dans un appareil de dissolution USP-2 muni de pale, on dissout respectivement de 7 à 25 %, de 45 à 70 % et d'au moins 96 % de médicament dans ledit acide chlorhydrique 0,1 N.

16. Composition pharmaceutique selon l'une quelconque des revendications 1 à 11, ou forme pharmaceutique selon l'une quelconque des revendications 12 à 15, destinée à être utilisée dans une méthode de thérapie ou de diagnostic du corps animal humain ou non humain.

17. Composition pharmaceutique selon l'une quelconque des revendications 1 à 11, destinée à être utilisée dans la fabrication d'une forme pharmaceutique à administrer par voie orale à un mammifère nécessitant le traitement, **caractérisée en ce que** ladite forme pharmaceutique peut être administrée à tout moment de la journée indépendamment de la prise de nourriture par ledit mammifère.

18. Utilisation d'une composition pharmaceutique selon l'une quelconque des revendications 1 à 11, pour la fabrication d'une forme pharmaceutique à administrer par voie orale à un mammifère nécessitant le traitement, **caractérisée en ce que** ladite forme pharmaceutique peut être administrée à tout moment de la journée indépendamment de la prise de nourriture par ledit mammifère.

19. Utilisation de la composition pharmaceutique selon l'une quelconque des revendications 1 à 11, pour la fabrication d'un médicament destiné à être utilisé dans une méthode de thérapie ou de diagnostic du corps humain ou non humain.

20. Trousse pharmaceutique convenant à la commercialisation, comprenant un récipient, une forme pharmaceutique à administrer par voie orale selon l'une quelconque des revendications 12 à 16, et, associé à ladite trousse, un document écrit non limité en ce qui est de savoir si la forme pharmaceutique peut être administrée avec ou sans la nourriture.
